# EUROPEAN PATENT APPLICATION

(11) **EP 4 730 747 A1**
(43) Date of publication of application: **22.04.2026**
(21) Application number: 25209318.2
(22) Date of filing: 17.10.2025
(51) Int. Cl.: H04L 65/1095, H04L 67/12, H04L 69/14

(54) **SYSTEMS AND METHODS FOR MULTI-LINK WIRELESS COMMUNICATION OF MEDICAL DATA**

(30) Priority: 18.10.2024 US 202463709004 P
(71) Applicant: Stryker Corporation, Portage, MI 49002 (US)
(72) Inventor: ANDRÉ, Marc, Portage, 49002 (US); FEINGOLD, Benjamin Hyman, Portage, 49002 (US)
(74) Representative: V.O.

(57) **Abstract**

A method for multi-link wireless transmission of medical images includes establishing a wireless communication connection between a medical data source and a medical data sink, the wireless communication connection comprising a first wireless communication link and a second wireless communication link; transmitting the medical images from the medical data source to the medical data sink via the wireless communication connection, wherein the medical images are transmitted via the first wireless communication link and the second wireless communication link; determining data loss over the first wireless communication link; and reconstructing at least one of the medical images using data transmitted via the second wireless communication link to correct for the data loss over the first wireless communication link.

## Description

### CROSS-REFERENCE TO RELATED APPLICATIONS

This application claims the benefit of U.S. Provisional Application No. 63/709,004, filed October 18, 2024, the entire contents of which are hereby incorporated by reference herein.

### FIELD

The present disclosure relates generally to medical device wireless communication and, more specifically, to wireless communication of medical data.

### BACKGROUND

Medical procedures often involve the use of one or more medical imaging devices for providing visualization of a region of interest of a patient to one or more users (e.g., one or more surgeons or other medical personnel). For example, an endoscopic camera can be used for providing visualization of a surgical cavity during a minimally invasive surgical procedure or an open-field camera may be used for fluorescence imaging of tissue of interest. Images generated by a medical imaging device (single snapshot images and/or video frames) may be transmitted to a display device for display to a user *(e.g.,* a surgeon or other medical personnel) and/or to an image processing device for analysis, augmentation, enhancement, other image processing, and/or display.

Medical imaging devices often generate high-definition images to capture small details of tissue of interest that may be relevant to treatment. Medical imaging devices are typically connected by high-data-rate cables to display devices and/or image processing devices, either directly or via one or more wired network components. Medical imaging devices are often mobile so that they can be moved to a suitable position within a medical room and/or moved between medical rooms as needed. The requirement for mobility, as well as the relatively long data cables used to provide mobility, often poses cable routing difficulties. These difficulties are exacerbated by the presence of other types of cables associated with the medical imaging devices and/or other devices in the area, such as power cables, control communication cables, fluid lines, etc.

Wireless communication technology is, of course, well known for reducing the need for data cables. However, the use of wireless communication for medical imaging, in particular high-definition medical imaging, poses a number of challenges, including the need for high data rates and low latency, that may be difficult for wireless communication technology to meet. This is particularly true with the increasing congestion of wireless communication bands resulting from the presence of multiple wireless communication devices within a given area.

### SUMMARY

According to various aspects, systems and methods for multi-link wireless communication of medical data, such as medical images, include transmitting medical data from a medical data source to a medical data sink over multiple links of a wireless communication connection between the medical data source and the medical data sink. The data for a given set of medical data (e.g., the data for a medical image, such as a single snapshot image or a video frame) can be sent over multiple wireless communication links such that the data sent over one of the links can be used to correct for loss or delay of data sent over another of the links that may occur, for example, due to wireless channel congestion and/or a drop in signal quality. By transmitting medical data using multiple links, the medical data can be reliably transmitted despite the loss or delay of data on one of the links. This multi-link communication of medical data can improve reliability of wireless communication of medical data and decrease latency, which may be particularly advantageous for wireless transmission of medical images.

According to an aspect, a method for multi-link wireless transmission of medical images includes establishing a wireless communication connection between a medical data source and a medical data sink, the wireless communication connection comprising a first wireless communication link and a second wireless communication link; transmitting the medical images from the medical data source to the medical data sink via the wireless communication connection, wherein the medical images are transmitted via the first wireless communication link and the second wireless communication link; determining data loss over the first wireless communication link; and reconstructing at least one of the medical images using data transmitted via the second wireless communication link to correct for the data loss over the first wireless communication link. A first data set associated with at least one of the medical images may be transmitted via the first wireless communication link, and a second data set associated with the at least one of the medical images may be transmitted via the second wireless communication link. The first and second data sets may be, or include, at least some of the same information. The first data set and the second data set may e.g. both include the same information (*i.e.,* two sets of the same data). The first and second data sets may include at least some different information.

At least a portion of data for the medical images that is transmitted via the first wireless communication link may be also transmitted via the second wireless communication link. Determining data loss over the first wireless communication link may include comparing data received at the medical data sink via the first wireless communication link to data received at the medical data sink via the second wireless communication link.

Determining data loss over the first wireless communication link may include analyzing data received via the first wireless communication link for indications of data loss. Reconstructing the at least one of the medical images may include reconstructing a first portion of the at least one of the medical images using data transmitted via the first wireless communication link and reconstructing a second portion of the at least one of the medical images using data transmitted via the second wireless communication link. Transmitting medical images from the medical data source to the medical data sink via the wireless communication connection may include transmitting the medical images at a higher fidelity via the first wireless communication link and transmitting the medical images at a lower fidelity via the second wireless communication link. Reconstructing the at least one of the medical images using data transmitted via the second wireless communication link to correct for the data loss over the first wireless communication link may include using an image transmitted via the second wireless communication link in place of an image affected by the data loss.

Transmitting the medical images from the medical data source to the medical data sink via the wireless communication connection may include transforming the medical images into higher fidelity information and lower fidelity information, transmitting both the higher fidelity information and the lower fidelity information via the first wireless communication link, and transmitting the higher fidelity information or the lower fidelity information, but not both, via the second communication link. The higher fidelity information may include higher frequency information, information compressed with a lower compression ratio, and/or higher resolution information, and the lower fidelity information may include lower frequency information, information compressed with a higher compression ratio, and/or lower resolution information. The higher fidelity information comprises at least one of luminance and chroma. The lower fidelity information may include at least one of luminance and chroma. The lower fidelity information may be transmitted via the second communication link.

The medical images may be video frames. The method may include determining that a current frame cannot be reconstructed due to data losses over the first and second wireless communication links and retrieving a previous frame from a frame buffer. The method may include dynamically adjusting a buffer size based on link quality of the first and second wireless communication links.

The method may include monitoring link quality of a plurality of wireless communication links that includes the first and second wireless communication links, and switching wireless communication links based on the link quality. The method may include redistributing data among the plurality of wireless communication links based on the link quality.

The medical data sink may include an image processing system. The image processing system may transmit the medical images via a wired connection to a display. The medical data source may include a medical imager.

According to an aspect, a system for multi-link wireless transmission of medical images includes a medical data source and a medical data sink, wherein the medical data source comprises one or more processors and memory storing instruction for execution by the one or more processors for causing the medical data source to: establish a wireless communication connection with the medical data sink, the wireless communication connection comprising a first wireless communication link and a second wireless communication link, and transmit the medical images from the medical data source to the medical data sink via the wireless communication connection, wherein the medical images are transmitted via the first wireless communication link and the second wireless communication link, and wherein the medical data sink comprises one or more processors and memory storing instruction for execution by the one or more processors for causing the medical data source to: determine data loss over the first wireless communication link, and reconstruct at least one of the medical images using data transmitted via the second wireless communication link to correct for the data loss over the first wireless communication link.

At least a portion of data for the medical images that is transmitted via the first wireless communication link may be also transmitted via the second wireless communication link. Determining data loss over the first wireless communication link may include comparing data received at the medical data sink via the first wireless communication link to data received at the medical data sink via the second wireless communication link. Determining data loss over the first wireless communication link may include analyzing data received via the first wireless communication link for indications of data loss. Reconstructing the at least one of the medical images may include reconstructing a first portion of the at least one of the medical images using data transmitted via the first wireless communication link and reconstructing a second portion of the at least one of the medical images using data transmitted via the second wireless communication link.

Transmitting medical images from the medical data source to the medical data sink via the wireless communication connection may include transmitting the medical images at a higher fidelity via the first wireless communication link and transmitting the medical images at a lower fidelity via the second wireless communication link. Reconstructing the at least one of the medical images using data transmitted via the second wireless communication link to correct for the data loss over the first wireless communication link may include using an image transmitted via the second wireless communication link in place of an image affected by the data loss.

Transmitting the medical images from the medical data source to the medical data sink via the wireless communication connection may include transforming the medical images into higher fidelity information and lower fidelity information, transmitting both the higher fidelity information and the lower fidelity information via the first wireless communication link, and transmitting the higher fidelity information or the lower fidelity information, but not both, via the second communication link. The higher fidelity information may include higher frequency information, information compressed with a lower compression ratio, and/or higher resolution information, and the lower fidelity information comprises lower frequency information, information compressed with a higher compression ratio, and/or lower resolution information. The higher fidelity information may include at least one of luminance and chroma. The lower fidelity information may include at least one of luminance and chroma. The lower fidelity information may be transmitted via the second communication link.

The medical images may be video frames. The memory of the medical data sink may store instruction for determining that a current frame cannot be reconstructed due to data losses over the first and second wireless communication links and retrieving a previous frame from a frame buffer. The memory of the medical data sink may store instruction for dynamically adjusting a buffer size based on link quality of the first and second wireless communication links.

The medical data source and medical data sink may be configured to monitor link quality of a plurality of wireless communication links that includes the first and second wireless communication links, and switch wireless communication links based on the link quality.

The medical data sink may include an image processing system. The image processing system may be configured to transmit the medical images via a wired connection to a display. The medical data source may include a medical imager.

According to an aspect, a non-transitory computer readable storage medium stores (or a computer program product comprises) instructions for execution by one or more processors of a computing system to cause the computing system to establish a wireless communication connection between a medical data source and a medical data sink, the wireless communication connection comprising a first wireless communication link and a second wireless communication link; transmit medical images from the medical data source to the medical data sink via the wireless communication connection, wherein the medical images are transmitted via the first wireless communication link and the second wireless communication link; determine data loss over the first wireless communication link; and reconstruct at least one of the medical images using data transmitted via the second wireless communication link to correct for the data loss over the first wireless communication link.

The transitory computer readable storage medium stores instructions for execution by one or more processors of a computing system to cause the computing system to perform any of the functions of the systems described above and/or performed any of the methods described above.

It will be appreciated that any of the variations, aspects, features, and options described in view of the systems applies equally to the methods and vice versa. It will also be clear that any one or more of the above variations, aspects, features, and options can be combined.

### BRIEF DESCRIPTION OF THE DRAWINGS

The invention will now be described, by way of example only, with reference to the accompanying drawings, in which:
FIG. 1 illustrates an exemplary medical room environment (e.g., a single medical room, a set of medical rooms, associated areas such as nurse's stations, etc.) that includes various devices capable of communicating wirelessly;
FIG. 2 illustrates an exemplary system that includes a medical data source configured for wirelessly transmitting medical data and a medical data sink configured for wirelessly receiving medical data;
FIG. 3 illustrates an example of the medical data source of FIG. 2 configured as a medical image source;
FIG. 4 illustrates aspects of the medical data sink of FIG. 2 configured to reconstruct medical images based on the multi-link data transmission from the medical data source of FIG. 3;
FIG. 5 illustrates an exemplary method for multi-link wireless transmission of medical data;
FIG. 6 illustrates an exemplary computing system; and
FIG. 7 illustrates an exemplary system in which a medical data source is configured to send command data to a medical data sink for controlling one or more functions of the medical data sink.

### DETAILED DESCRIPTION

Reference will now be made in detail to implementations and examples of various aspects and variations of the invention, examples of which are illustrated in the accompanying drawings. Various devices, systems, and methods are described herein. Although at least two variations of the devices, systems, and methods are described, other variations may include aspects of the devices, systems, and methods described herein combined in any suitable manner having combinations of all or some of the aspects described. Examples will now be described more fully hereinafter with reference to the accompanying drawings; however, they may be embodied in different forms and should not be construed as limited to the examples set forth herein. Rather, these examples are provided so that this disclosure will be thorough and complete, and will fully convey exemplary implementations to those skilled in the art.

According to various aspects, systems, devices, and methods include transmitting medical data, such as a medical image, from a medical data source to a medical data sink over multiple links of a wireless communication connection. The data transmitted via one link can be used to correct for loss or delay of data transmitted via another link. This redundancy can increase the reliability of wireless transmission of the medical data and/or decrease latency, which may be particularly advantageous for wirelessly transmitting medical images, including high-definition and ultra-high-definition images.

A medical data source and a medical data sink may each be configured to establish a multi-link wireless communication connection with each other. The links can be on different wireless bands, and/or on different channels of the same band, and/or on different wireless protocols. The medical data source and the medical data sink may be configured to monitor link quality and make adjustments as necessary, such as by changing the channel used for a given link and/or establishing new links and breaking poor performing links. The medical data source and a medical data sink may dynamically adjust how medical data is communicated via the links based on changes in link quality. For example, a drop in quality of a given link may lead to an increase in compression ratio used for the data of that link.

A medical data source, such as a medical imager, may transmit a set of medical data over multiple links, for example, by sending duplicate data over the multiple links. On the receiving end, the medical data sink, such as a display or image processing system, may reconstruct the set of medical data by replacing data lost or delayed on one link with data received on another link in a one-to-one fashion. Alternatively, the data sent on each of multiple links may be different. For example, higher-fidelity data for a given set of medical data requiring a higher-quality link (e.g., higher bandwidth, better signal strength, etc.) may be sent on a higher-quality link and lower-fidelity data for the given set of medical data may be sent on a lower-quality link. The medical data sink may use the higher-quality link as a primary source of data in order to reconstruct the medical data with the higher fidelity, but if there should be data loss or delay on the higher-quality link, the medical data sink may use the lower-fidelity data received via the lower-quality link to reconstruct some or all of the set of data. While this may result in a reduction in quality of some or all of the set of data, the quality reduction may be acceptable (e.g., the reduction in resolution of a medical image may not be perceivable to a user).

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise, as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure, in some aspects, also relates to devices or systems for performing the operations herein. The devices or systems may be specially constructed for the required purposes, may comprise a general-purpose computer selectively activated or reconfigured by a computer program stored in the computer, or may include any combination thereof. Computer instructions for performing the operations herein can be stored in any combination of non-transitory, computer-readable storage media, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, or any type of media suitable for storing electronic instructions, and each coupled to a computer system bus. One or more instructions for performing the operations herein may be implemented in or executed by one or more Application Specific Integrated Circuits (ASICs), Field Programmable Gate Arrays (FPGAs), Digital Signal Processing units (DSPs), Graphics Processing Units (GPUs), or Central Processing Units (CPUs). Furthermore, the computers referred to herein may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

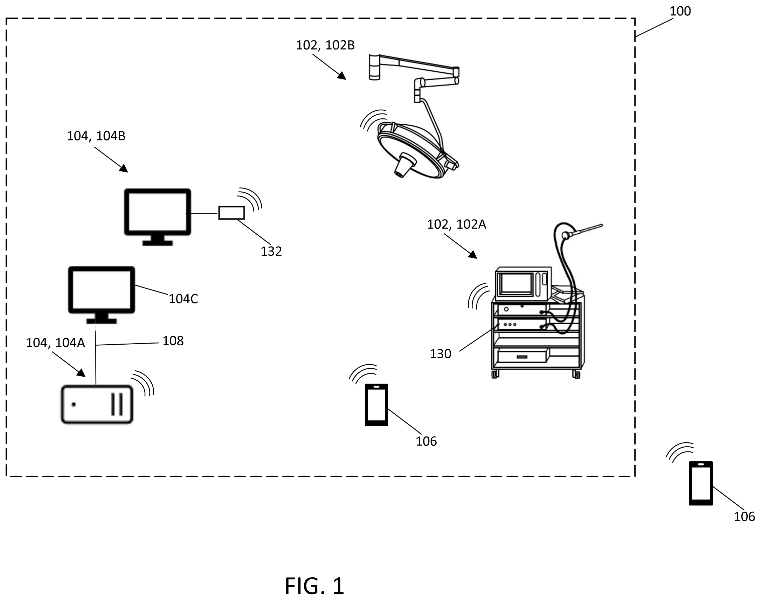

FIG. 1 illustrates an exemplary medical room environment 100 *(e.g.,* a single medical room, a set of medical rooms, associated areas such as nurse's stations, etc.) that includes various devices capable of communicating wirelessly. At least one of the devices may generate medical data, in which case the devices are medical data sources 102. At least one of the devices may use medical data received from a medical data source 102, in which case the device is a medical data sink 104. Some devices may both generate and receive medical data, in which case the device is both a medical data source 102 and a medical data sink 104. One or more of the medical data sources 102 and medical data sinks 104 may be capable of communicating wirelessly and, as such, may establish wireless communication connections using available wireless communication bands, such as the 2.4 GHz, 5 GHz, and/or 6 GHz bands commonly used for Wireless Fidelity (WiFi) communication. The wireless data communicated between medical data sources 102 and medical data sinks 104 may be encoded, for example, by Orthogonal Frequency Division Multiplexing (OFDM), Complementary Code Keying (CCK), Quadrature Amplitude Modulation (QAM), Phase-Shift Keying (PSK) (including binary PSK and orthogonal PSK), or any other method that allows transmitting with a required data rate and within the available channel bandwidth. Wireless data may be communicated between multiple medical data sources 102 and multiple medical data sinks 104 within a multi-input, multi-output (MIMO) framework.

A medical data source 102 may be any medical device or combination of devices that generates data in the medical room environment 100. For example, a medical data source 102 may be a medical imager 102A (for example, a C-arm fluoroscopic imager, an open-field imager, or an endoscopic imager) that generates images (as used herein, the term "image" is inclusive of single snapshot images and video frames). A medical data source 102 may be a device that includes components for generating medical images, such as a surgical light 102B that includes an in-light camera. As used herein, a "medical data source" is any device or combination of devices present in the medical room environment 100 that generates data that is transmitted to another device, and "medical data" is any data generated by a medical data source. Thus, for example, a medical data source 102 may be a pan-tilt-zoom (PTZ) camera used for observation of the medical room environment but not specifically for providing medical care. While it may be used to generate medically relevant data (data that may be used in providing medical treatment), a PTZ camera may also be used to generate non-medically relevant data, such as a video stream of a medical room (e.g., during set-up, cleaning, etc.). A medical data source 102 may be a keyboard, video, and mouse (KVM) source, such as a workstation in the medical room that allows users (e.g., surgeons, medical staff, and the like) to control the various other devices in the medical room for a variety of purposes. For example, a camera may lose focus and instead of a user directly adjusting the focus, which may compromise sterilization, a user may adjust the focus remotely through the KVM source. A medical data source 102 may be, or include, any user input device configured for a user to provide a user input, such as user commands and user selections, and can include a foot pedal, a hand-held controller, and a microphone device for receiving user voice commands. As such, data generated by a medical data source 102 may include command data. A medical data source 102 may be a system that provides status information (e.g., a system operational parameter setting, an error notification, or an alarm notification) or other types of non-image information to a medical data sink 104 for display by the medical data sink 104 (or display device connected to the medical data sink 104). One or more of the medical data sources 102 may be mobile such that the medical data source 102 can be moved within the medical room environment 100 and/or moved to another medical room environment. For example, the medical imager 102A may be on a mobile cart that enables the medical imager 102A to be repositioned with a treatment room as desired by a user and/or moved to a different treatment room as needed.

One or more of the medical data sources 102 may include a wireless transceiver that enables the medical data source to communicate wirelessly with one or more medical data sinks 104. The wireless transceiver may be integral to the same component that outputs the medical data. For example, a wireless transceiver may be integral to a camera control unit 130 of medical imager 102A. Alternatively, the wireless transceiver may be provided by a peripheral device to which the component that generates the medical data is connected. For example, the camera control unit 130 may be connected to a standalone wireless transceiver.

Medical data sinks 104 may include any device capable of using data generated by a medical data source 102. For example, the medical data sinks 104 may include an image processing system 104A that processes images, such as for analysis, augmentation, enhancement, display, routing to other medical data sinks 104, and/or any other purpose. The medical data sinks 104 may include one or more displays 104B for displaying medical data generated by one or more of the medical data sources 102, such as user interfaces (e.g., including status information such as system operational parameter settings, error notifications, and alarm notifications), single snapshot images, and/or video feeds. Other examples of medical data sinks 104 include, but are not limited to, data recorders, projectors, and virtual or augmented reality headsets. A medical data sink 104 can be a device that receives control commands from a medical data source 102. For example, a medical data source 102 can be a user control device (e.g., a foot pedal or a hand-held remote control), and the medical data sink 104 can be (or include) a device controlled by the user control device (e.g., a surgical instrument, such as a shaver).

One or more medical data sinks 104 may include a wireless transceiver that enables the medical data source to communicate wirelessly with one or more medical data sources 102 and/or one or more other medical data sinks 104. The wireless transceiver may be integral to the same component that uses the medical data (e.g., integral to the display 104B) or may be provided by a peripheral component connected to the component that uses the medical data *(e.g.,* display 104B may be connected to a wireless access point 132). One or more medical data sinks 104 may not be capable of wireless communication and may be communicatively connected to another medical data sink 104 via a wired connection for receiving medical data. For example, image processing system 104A may receive medical images from medical imager 102A and may transmit the medical images to a display 104C via a wired connection 108.

One or more of the medical data sinks 104 may be mobile such that the medical data sink 104 can be moved within the medical room environment 100 and/or moved to another medical room environment. For example, the image processing system 104A may be on a mobile cart that enables the image processing system 104A to be repositioned within a treatment room as desired by a user and/or moved to a different treatment room as needed.

As noted above, an example of a medical data sink 104 is an image processing system 104A. Image processing system 104A may be configured as a central hub or controller that may control the generation, transmission, and/or use of medical data by one or more of the medical data sources 102 and medical data sinks 104 and/or may process medical data generated by one or more of the medical data sources 102 (either for its own use or for providing to another medical data sink 104). Image processing system 104A may route medical data to one or more medical data sinks 104, such as wirelessly to a medical data sink 104 capable of wireless communication or via a wired connection or wired network to a medical data sink 104 that is not capable of wireless communication. For example, image processing system 104A may receive video from medical imager 102A and transmit the video (with or without first analyzing, augmenting, enhancing, or otherwise processing the video) to display 104B wirelessly to a non-wireless display via a suitable wired connection. Image processing system 104A may generate medical data for transmission to another device and use medical data received from another device and, thus, may be both a medical data source and a medical data sink.

One or more devices 106 that generate electromagnetic energy in the same waveband(s) as used by one or more medical data sources 102 and medical data sinks 104 may be in the vicinity of the medical room environment 100. The devices 106 may generate wireless signals that are within range of the medical data sources 102 and medical data sinks 104 and may share one or more of the same wireless bands as the medical data sources 102 and medical data sinks 104. Devices 106 may be within the same medical room environment 100 or may be outside of the medical room environment 100. Devices 106 may include, for example, personal smartphones of medical personnel in the vicinity of the medical room environment or other types of wireless devices that are connected to one or more WiFi networks that at least partially cover the medical room environment 100, may include short-range wireless communication devices (e.g., Bluetooth devices), such as wireless controllers and wireless headsets, and/or may include devices that generate non-communication electromagnetic radiation in the same band(s) used by the medical data sources 102 and medical data sinks 104 *(e.g.,* devices that generate microwave radiation). The presence of devices 106 may cause wireless congestion and/or interference in one or more of the wireless communication bands or channels used by the medical data sources 102 and medical data sinks 104.

Certain medical data may require high data rates for transmission from a medical data source 102 to a medical data sink 104. For example, high-definition or ultra-high-definition images (e.g., 720p, 1080p, 2160p, 4320p, and higher resolutions) may be transmitted at a video frame rate *(e.g.,* 24, 30, 60, 120, or higher frames per second). The quality of transmission of such medical data may be important to a medical treatment. For example, the quality of endoscopic video generated and wirelessly transmitted by medical imager 102A and received at display 104B for display to a user *(e.g.,* a surgeon) may affect the ability of the user to visualize the surgical cavity while manipulating tissue within the surgical cavity. As such, interruption to the wireless transmission of medical data or degradation to the medical data during wireless transmission that may result from congestion or wireless signal degradation may adversely affect medical treatment. As described below, the medical data sources 102 and medical data sinks 104 may be configured to communicate medical data over multi-link connections in which data transmitted on one link can be used to correct for loss or delay of data transmitted on another link, which may provide high reliability and/or low latency. The multi-link communication described within can enable reliable wireless communication of medical data at high data rates within a medical room environment 100 that may have high congestion and/or wireless signal interference of one or more wireless communication bands or channels used by the medical data sources 102 and medical data sinks 104.

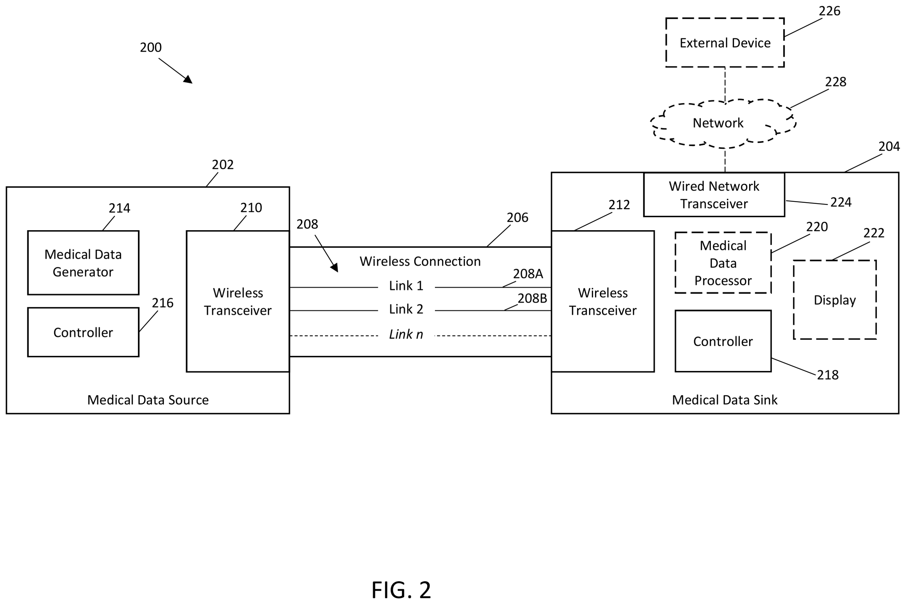

FIG. 2 illustrates an exemplary system 200 that includes a medical data source 202 configured for wirelessly transmitting medical data and a medical data sink 204 configured for wirelessly receiving medical data. The components illustrated in FIG. 2 are functional components that may be implemented by any suitable combination of hardware and software. Medical data source 202 may be any of the medical data sources 102 of FIG. 1, and medical data sink 204 may be any of the medical data sinks 104 of FIG. 1. Medical data source 202 and medical data sink 204 may establish a multi-link wireless communication connection 206 that includes multiple links 208. The wireless communication connection 206 between the medical data source 202 and the medical data sink 204 may include any number of links 208, such as two links, three links, four links, five links, six links, etc. The links 208 may be on different channels of the same wireless band and/or may be on different wireless bands. For example, link 208A may be on a first channel of the 5.0 GHz band and link 208B may be on a second channel of the 5.0 GHz band or on a channel of the 6.0 GHz band.

Medical data source 202 includes a wireless transceiver 210 and medical data sink 204 includes a wireless transceiver 212. Wireless transceiver 210 and wireless transceiver 212 are configured to establish and maintain the wireless communication connection 206, including establishing, maintaining, and/or breaking the links 208, and to convert data into data packets for transmitting via the links 208 and/or assemble data packets received via the links 208 into useable information. Wireless transceivers 210 and 212 may communicate information with each other for maintaining the wireless communication connection 206, including information associated with link quality, such as signal strength, noise, signal-to-noise ratio, channel congestion, data loss, data rate capability, and/or any other information useful for maintaining the wireless communication connection 206 and transmitting data via the wireless communication connection 206. For example, wireless transceiver 210 may inform wireless transceiver 212 about link quality and wireless transceiver 212 may act on the link quality as needed (e.g., changing channel, changing bandwidth, changing encoding, etc.), and/or wireless transceiver 212 may inform wireless transceiver 210 about link quality and wireless transceiver 210 may act on the link quality as needed (e.g., changing channel, changing bandwidth, changing encoding, etc.). As such, the decision regarding how to act on link quality information can be made by medical data source 202 or medical data sink 204.

Wireless transceivers 210 and 212 may adjust attributes of the links 208 over time. For example, wireless transceivers 210 and 212 may switch a link 208 from one channel to another, such as when a channel becomes over-congested and/or has poor signal characteristics. Wireless transceivers 210 and 212 may add one or more new links 208 and may break one or more previously established links 208. The wireless transceivers 210 and 212 may dynamically distribute traffic over the links 208 based on link characteristics. For example, if the quality of one link reduces *(e.g.,* falls below a predetermined threshold), some or all of the traffic may be shifted to one or more other links

Medical data source 202 includes a medical data generator 214 configured to generate medical data for transmitting via the wireless communication connection 206. The medical data generator 214 may be, for example, an imaging sensor and associated components for generating medical images. The medical data generator 214 and the wireless transceiver 210 may be components of the same device or may be components of different devices that are connected together. For example, the wireless transceiver 210 may be integrated into a medical imager, or the wireless transceiver 210 may be a component of a separate device (e.g., a wireless access point) to which the medical imager is communicatively connected. As used herein, a medical data source refers to a device that has integrated wireless communication capability *(e.g.,* an integrated wireless transceiver) and refers to a combination of a device that generates medical data and a wireless transceiver to which the medical data generating device is connected.

The medical data source 202 includes a controller 216 for controlling the transmission of medical data generated by the medical data generator 214 and transmitted by the wireless transceiver 210. The controller 216 may be a functional component implemented by one or more processors that provide other functionality of the medical data source 202. For example, one or more processors used for generating medical data *(e.g.,* one or more processors of medical data generator 214) may provide some or all of the functionality of controller 216. Controller 216 may be implemented as a discrete computing unit that includes one or more processors that execute one or more programs for achieving the functionality of the controller 216 described herein.

The controller 216 controls aspects of how the medical data is transmitted using the multiple links 208. The controller 216 may receive medical data from the medical data generator 214 and process the medical data such that the medical data can be transmitted over multiple links 208 in a way that enables the medical data sink 204 to reliably receive the medical data in the presence of high wireless congestion and/or wireless signal interference. For example, the controller 216 may convert a medical image into a higher-fidelity representation and a lower-fidelity representation and control the wireless transceiver 210 to send the higher-fidelity representation via a primary link *(e.g.,* link 208A) and the lower-fidelity representation via a secondary link *(e.g.,* link 208B). The primary link may be the link having a higher quality *(e.g.,* a higher-data-rate capability and/or higher reliability) than the secondary link.

Turning to the medical data sink 204, the medical data sink 204 includes a controller 218 that controls the reconstruction of medical data received via the wireless communication connection 206. The controller 218 may be a functional component implemented by one or more processors that provide other functionality of the medical data sink 204. For example, one or more processors that process medical data once reconstructed *(e.g.,* an image processor that analyzes, augments, enhances, or otherwise manipulates medical images) may provide some or all of the functionality of controller 218. Controller 218 may be implemented as a discrete computing unit that includes one or more processors that execute one or more programs for achieving the functionality of the controller 218 described herein.

The controller 218 may use data received via different links 208 in reconstructing a particular set of the medical data. Continuing with the example above, the controller 218 may reconstruct a medical image using just the data received via the primary link *(e.g.,* the higher-fidelity representation received via link 208A), just the data received via the secondary link *(e.g.,* the lower-fidelity representation received via link 208B), and/or data from both the primary link and the secondary link. As discussed further below, the controller 218 may reconstruct a set of medical data *(e.g.,* a medical image) using data transmitted via one link 208 to correct for data loss or delay over another link 208 *(e.g.,* data loss or delay caused by increased wireless band congestion and/or signal interference).

The medical data sink 204 may include a medical data processor 220 that may process the medical data after it has been reconstructed. For example, the medical data processor 220 may analyze, augment, enhance, or otherwise manipulate or use the medical data, and/or may cause display of the medical data (with or without first manipulating the medical data) on a display 222 of the medical data sink 204. In examples in which the medical data includes command data, the medical data processor 220 may perform one or more functions according to the command data. For example, the medical data sink 204 may be a surgical instrument system (e.g., a pump system, a cutter system, an imaging system, etc.) that is remotely controllable by the medical data source 202 (e.g., a user control device, such as a foot pedal or hand-held controller), and the medical data processor 220 may perform one or more surgical instrument control functions in accordance with the command data (e.g., activate a surgical instrument, change a mode or settings of the surgical instrument, and/or adjust at least one operational parameter of the surgical instrument).

The medical data sink 204 may include a wired network transceiver 224 or other type of wired communication component that may be used for transmitting the medical data (with or without processing by the medical data processor 220) to one or more external devices 226 *(e.g.,* an external display or a medical data storage device) via a suitable communication connection (e.g., directly via a video cable or via a network 228). Some or all of the functionalities of medical data processor 220 and the controller 218 may be implemented by the same hardware processor or set of hardware processors. For example, a CPU may be configured to reconstruct medical data received by wireless transceiver 212, provide the reconstructed medical data to a GPU for enhancement, receive the enhanced medical data from the GPU, and control display of the enhanced medical data and/or transmission of the enhanced medical data to an external device 226.

The medical data sink 204 may be a device that has integrated wireless communication capability *(e.g.,* a display with an integrated wireless network adapter). The medical data sink 204 may be a combination of a device that consumes medical data and an external wireless transceiver to which the medical data consuming device is connected *(e.g.,* a display connected to a wireless access point).

The controller 216 of medical data source 202 and the controller 218 of medical data sink 204 may implement a network management tool *(e.g.,* a network management software application) that manages various aspects of the wireless communication connection 206. For example, a network management tool may be used to monitor link performance, adjust link configurations, adjust how links are used to transmit medical data, etc. A network management tool may use information regarding links obtained by wireless transceivers 210, 212 to manage the links 208. The network management tool of the medical data source 202 may send medical data reconstruction information to the network management tool of the medical data sink 204 *(e.g.,* over one or more of the links used to send the medical data and/or over a link not used to send the medical data) to inform the medical data sink how to use the data sent via the multiple links 208 to reconstruct medical data. For example, the network management tool of the medical data source 202 may send instructions to the medical data sink 204 indicating that link 208A is the primary link and link 208B is a secondary link. This may indicate to the medical data sink 204 to use data received via link 208A to reconstruct the medical data unless there is data loss on link 208A (in which case medical data sink 204 should use data received via link 208B to correct for the data loss). Optionally, data reconstruction information may be embedded in one or more of the links (e.g., link 208A and/or link 208B).

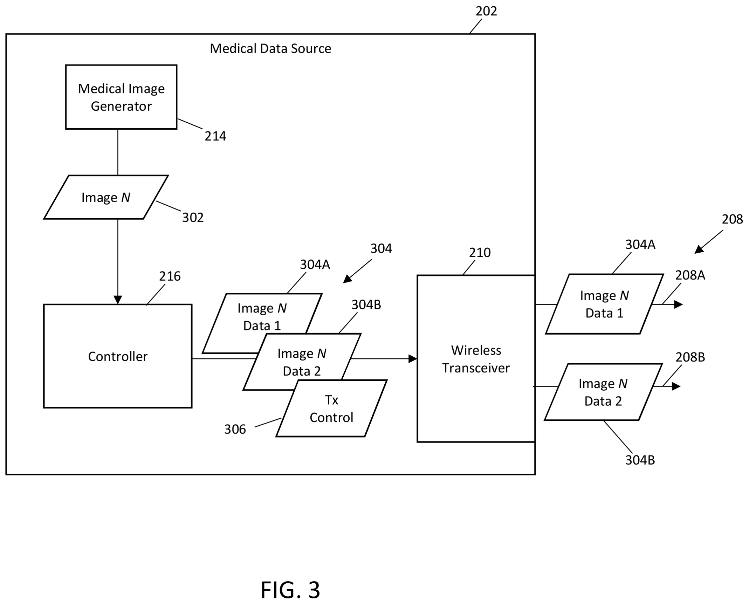

As noted above, the controller 216 of medical data source 202 may be configured for controlling transmission of medical data over multiple links 208 in a way that enables the medical data sink 204 to reliably receive the medical data in the presence of high wireless congestion and/or wireless signal interference. FIG. 3 illustrates an example of the medical data source 202 configured as a medical image source that generates medical images (e.g., high-definition images) and transmits the medical images *(e.g.,* at a video frame rate) over a multi-link connection with the medical data sink 204 in a way that enables the medical data sink 204 to reliably reconstruct the medical images in the presence of high wireless congestion and/or wireless signal interference.

The controller 216 may receive an image 302 from the medical data generator 214 and may generate multiple sets of data 304 from the medical image 302. In the illustrated example, the controller 216 generates a first data set 304A associated with the medical image and a second data set 304B associated with the medical image 302. The sets of data 304 generated from the medical image 302 may be, or include, at least some of the same information. For example, the first data set 304A and the second data set 304B may both include the same information (*i.e.,* two sets of the same data) such that the medical image 302 may be reconstructed by the medical data sink 204 using either the first data set 304A or the second data set 304B depending on whether each data set 304A, 304B is received by the medical data sink 204. Alternatively, the sets of data 304 generated from the medical image 302 may include at least some different information. For example, the first data set 304A may include a lower-fidelity representation of the image 302 *(e.g.,* a lower-resolution version of the image 302) and the second set of data 304B may include a higher-fidelity representation of the image 302 *(e.g.,* a higher-resolution version of the image 302), or the first data set 304A may include a base image comprised of low frequency aspects of the image and the second set of data 304B may include the base image and high frequency aspects of the image that, together with the base image, can be used to reconstruct the original image or a higher-fidelity version of the image than the base image.

The controller 216 may provide the different sets of data 304 for the medical image 302 to the wireless transceiver 210, which may transmit the different sets of data 304 using different links 208 of the wireless communication connection 206. For example, the wireless transceiver 210 may transmit the first data set 304A via link 208A and may transmit the second data set 304B via link 208B. The controller 216 may provide transmission control information 306 to the wireless transceiver 210 to control how the wireless transceiver 210 handles the different sets of information. The control information 306 may indicate which link the wireless transceiver 210 should use to send a given set of data 304. For example, the control information 306 may indicate that the first data set 304A should be transmitted via link 208A and the second data set 304B should be transmitted via link 208B. Alternatively, the control information 306 may provide qualitative instructions that the wireless transceiver 210 may use to determine which link 208 to use for which set of data 304. For example, the control information 306 may indicate that the first data set 304A should be transmitted via a higher-quality link and the second data set 304B may be transmitted via a lower-quality link, and wireless transceiver 210 may select which link 208 to use for a given set of data based on its knowledge of link quality. Wireless transceiver 210 may communicate link information to the controller 216 to enable the controller 216 to generate the control information 306. For example, wireless transceiver 210 may provide the controller 216 a list of available links 208 and information about the links *(e.g.,* one or more indicators of quality, such as signal strength, data rate, band and/or bandwidth, data loss statistics, etc.).

The wireless transceiver 210 may switch which links 208 are used for sending the sets of data 304 and/or may change one or more attributes of one or more of the links 208 based, for example, on quality characteristics of the links 208. For example, the wireless transceiver 210 may respond to a drop in quality *(e.g.,* a drop in quality below a predetermined threshold) of a link by establishing a new link and may then send data over the new link. Additionally, or alternatively, wireless transceiver 210 may change the channel and/or band of a given link 208 in response to a drop in link quality. The wireless transceiver may encode data sent via any of the links 208 in a way that a loss or delay of a certain amount or certain type of packets is acceptable *(e.g.,* the effect of the loss or delay of the packets is not significantly noticeable to the user). This may be achieved with forward error correction or by encoding the information of a medical image 302 across multiple packets in a way that the loss or delay of, for example, a single packet only has marginal impact on the image reconstruction by the medical data sink 204. The amount of, for example, forward error correction may be dynamically adjusted based on link quality and/or in concert with how the sets of data 304 are generated. For example, the controller 216 may dynamically increase the amount of forward error correction used for a link 208 in response to a drop in quality of the link 208 and the fidelity of the set of data 304 sent via the link 208 may be reduced to account for the increased amount of forward error correction and/or the decreased bandwidth of the link 208.

The controller 216 may generate the sets of data 304 for a given medical image 302 in a number of different ways. Optionally, the controller 216 may generate duplicate sets of data for the medical image 302 and may control wireless transceiver 210 such that each of the duplicate sets of data is transmitted via a different link 208. This redundancy may enable the medical data sink 204 to reconstruct the medical image 302 even when there is data loss or delay on one of the links 208.

In some examples, the duplicate sets of data transmitted via the different links each include the full-fidelity medical image 302 so that the medical data sink 204 can reconstruct the full-fidelity medical image 302 from the data received on either link 208 (should there be no loss or delay in data during transmission on that link). If there is loss or delay in data during transmission on one of the links 208, corresponding data received via the other link 208 can be used by the medical data sink 204 to replace the lost or delayed data without affecting the full-fidelity reconstruction. This can include replacing data for just one individual pixel, replacing data for one or more lines of pixels, replacing data for a region of multiple pixels, replacing data for one or more color channels of the medical image 302, and/or replacing all data for the medical image 302.

In some examples, the controller 216 may process the medical image 302 to generate higher-fidelity information *(e.g.,* a higher-resolution image and/or lower compression ratio) and lower-fidelity information (e.g., a lower-resolution image and/or a higher compression ratio) for the medical image 302 and may control the wireless transceiver 210 to send the higher-fidelity information on a primary link *(e.g.,* on link 208A) and the lower-fidelity information on a secondary link *(e.g.,* on link 208B). The medical data sink 204 may use the data received via the primary link *(e.g.,* the higher-fidelity information sent via link 208A) to reconstruct the medical image 302 if there is no loss or delay (or an acceptable amount of loss or delay) on the primary link. In the event there is loss or delay (or an unacceptably high amount of loss or delay) on the primary link, the medical data sink 204 may use at least some of the data from a secondary link *(e.g.,* the lower-fidelity information sent via link 208B) to reconstruct the medical image 302. For example, higher-fidelity information for one or more lines (or other regions) of the medical image 302 that have been lost or delayed may be replaced with lower-fidelity information for those one or more lines. Alternatively, the medical image 302 may be reconstructed entirely from the lower-fidelity information. The higher-fidelity information may be transmitted via a link 208 that has the higher data rate and/or quality, with the lower-fidelity information being transmitted via a link 208 with a lower data rate and/or quality. Attributes of the medical image 302 (or a set of medical images) that may be represented in higher-fidelity information and in lower-fidelity information may include different color channel values, luminance values, and chroma values. Different fidelities can encompass different compression ratios, different resolutions, different spatial frequencies, and/or different temporal resolutions.

The controller 216 may process the medical image 302 to generate a set of primary information that can be used by the medical data sink 204 to reconstruct the medical image 302 at an acceptable level of quality and a set of secondary information that can be used in combination with the primary information for reconstructing the medical image 302 with higher quality. Both the primary and secondary information may be transmitted via, for example, link 208A (if link 208A is the higher-quality link), and the primary information, but not the secondary information, may be transmitted via link 208B. The medical data sink 204 may seek to reconstruct a medical image 302 using the primary and secondary information received via link 208A so as to produce a higher-quality image. In the event that there is data loss or delay on link 208A, the medical data sink 204 may reconstruct at least a portion of the medical image 302 using the primary information sent via link 208B, resulting in a lower-quality (but still acceptable) image or portion of an image. The primary information can include, for example, a more perceivable aspect of medical image 302, and the secondary information can include, for example, a less perceivable aspect of medical image 302. For example, the primary information sent via link 208A can include a high-fidelity luminance channel of the medical image 302 and the secondary information can include high-fidelity chroma channels of the medical image 302. The set of data sent via link 208B may include the high-fidelity luminance channel but not the high-fidelity chroma channels. Optionally, lower-fidelity chroma channels may be included in the set of data sent via link 208B.

The fidelity of information transmitted on each link 208 may be dynamically adjusted based on link quality. For example, the fidelity *(e.g.,* the resolution) of higher-fidelity information sent on link 208A may be adjustable based on the quality of link 208A, and the fidelity of the lower-fidelity information sent on link 208B may be adjustable based on the quality of link 208B. In some examples, the fidelity of the information transmitted on each link 208 may be the same if, for example, the quality of each link is the same.

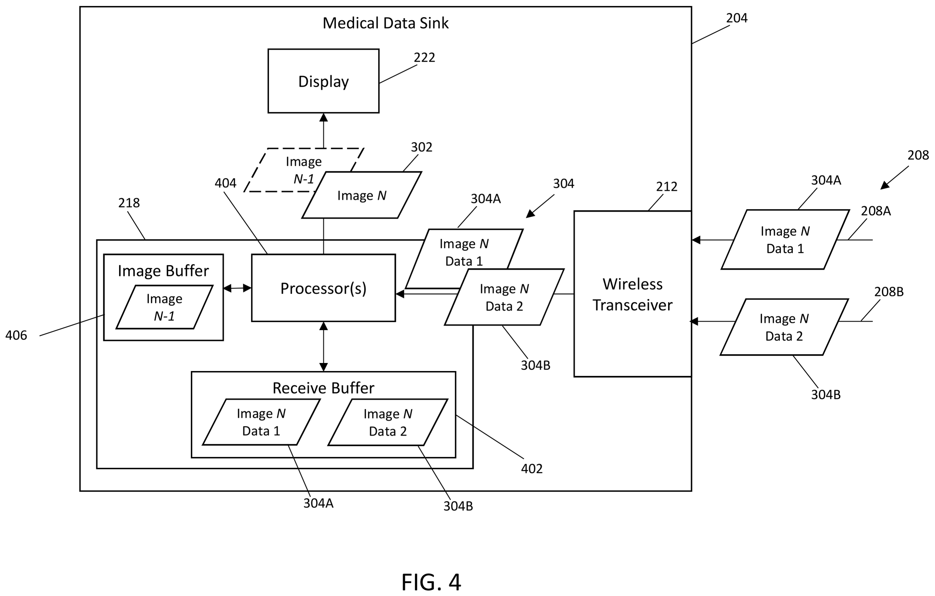

FIG. 4 illustrates aspects of medical data sink 204 configured to reconstruct medical images based on the multi-link data transmission from medical data source 202 of FIG. 3. The wireless transceiver 212 receives the sets of data 304 transmitted by the medical data source 202 via the links 208 in the form of data packets and assembles the data packets into the sets of data 304. The wireless transceiver 212 provides the sets of data 304 to the controller 218 of the medical data sink 204. The controller 218 uses data from one or more of the sets of data 304 to reconstruct the medical image 302.

The controller 218 and/or wireless transceiver 212 may determine data loss or delay on one of the links 208 and may use data received via a different link 208 to correct for the data loss or delay. For example, controller 218 may determine that data set 304A sent via link 208A *(e.g.,* which may be the primary link) is insufficient in some way to reconstruct medical image 302 and may use data from data set 304B sent via link 208B *(e.g.,* which may be a secondary link) to reconstruct medical image 302. If only some of data set 304A was lost or delayed, controller 218 may use data from data set 304B to replace only the lost or delayed data while still using the data from data set 304A that was received. For example, if data set 304A lacks a particular line of the image 302, that particular line of the image 302 may be reconstructed with the corresponding data from data set 304B.

Various characteristics of the medical image 302 that the controller 218 reconstructs may depend on how the medical image 302 is reconstructed. If there is no data loss or delay, the medical image 302 may be reconstructed from data sent via a primary link only, in which case the medical image 302 may be the best quality possible, given how the medical image 302 was transmitted. If there is data loss or delay on the primary link, data from the secondary link may be sufficient to replace the lost or delayed data without affecting image quality. For example, if a duplicate of the medical image 302 is sent on both the primary link and a secondary link, data from the secondary link may be used to replace data lost or delayed on the primary link without loss of fidelity. In some examples, however, the data sent on the secondary link does not include all of the information provided on the primary link. For example, the data sent via the primary link may include lower-fidelity information and higher-fidelity information that the controller 218 may use to reconstruct the medical image 302 at full fidelity, but the data sent via the secondary link may include only the lower-fidelity information. This reduction in fidelity may affect only a portion of the medical image 302 *(e.g.,* the portion affected by the data loss) when, for example, low-fidelity data from the secondary link is used for only a portion of the medical image 302. Alternatively, the reduction in fidelity may be manifested in the entire medical image 302 when, for example, the medical image is constructed entirely from the data received via the lower-fidelity link.

The controller 218 may determine data loss or delay by comparing data from data set 304A with data from data set 304B. For example, controller 218 may include a receive buffer 402 for storing data from the multiple links 208 as the data is received. One or more processors 404 of the controller 218 may periodically retrieve the buffered data and compare the data received via one link with the data received via another link to determine whether data has been lost or delayed. For example, the one or more processors 404 may compare some or all of data set 304A with some or all of data set 304B and determine that, for example, one or more lines of pixel values in data set 304B are not in data set 304A.

The receive buffer 402 may be sized for buffering at a sub-image level, such as a few lines of an image. The size of the buffer 402 may be dynamically adjusted based on quality aspects of the links 208. For example, the size of the receive buffer 402 may be increased when the quality of one or more of the links 208 drops below a threshold quality. The size of the receive buffer 402 may be changed, therefore, when poor-quality links are replaced with higher-quality links and/or when the quality of a link is improved. The size of the receive buffer 402 may be maintained at a minimum level given the link quality in order to minimize latency.

The controller 218 and/or wireless transceiver 212 may determine data loss or delay without comparing the sets of data 304. The controller 218 may analyze data received via each link 208 for indications of data loss or delay. For example, the controller 218 may calculate check values from received data (e.g., checksums) and compare the check values to the check values in the received data. The controller 218 may determine a loss or delay in data when the check values do not match. For example, the controller 218 may use an error-detecting algorithm such as a cyclic redundancy check. Additionally, or alternatively, the controller 218 may have some expectation of when data should be received (e.g., based on a pattern of data packet reception frequency) and may determine data loss or delay when the data is not received according to the expectation. Optionally, the controller 218 may utilize a packet counter and may determine a loss or delay of data when a packet is skipped. The controller 218 may receive packets at known intervals (often referred to as "keep-alive packets"), and may determine loss or delay of data when a packet is not received at the expected interval (this may be particularly useful when the data is control data).

The controller 218 may maintain an image buffer 406 (also referred to herein as a frame buffer) that may be used when a given image *(e.g.,* a video frame) cannot be reconstructed from the data received via the multiple links 208 (or cannot be reconstructed in time *(e.g.,* in time to meet a given frame rate of a video)). For example, if there is data loss or delay on both link 208A and link 208B that prevents reconstruction of image *N*(*e.g.,* a frame of a video), the one or more processors 404 may retrieve a previous image *N-1 (e.g.,* a previous video frame) from the image buffer 406 and use it in place of image N for displaying via display 222. The size of the image buffer 406 may be fixed *(e.g.,* a fixed number of images may be stored in the image buffer 406) or the size of the image buffer 406 may be dynamically adjustable based, for example, on a quality of one or more of the links 208. For example, the size of the image buffer 406 may be increased when the quality of the links 208 decreases *(e.g.,* below a certain threshold value).

The controller 218 and/or wireless transceiver 212 may transmit information to the medical data source 202 regarding link quality and/or data loss. For example, if the controller 218 uses data from a secondary link to correct for data loss or delay on a primary link, the controller 218 may send information to the medical data source 202 indicating that data loss or delay on the primary link required use of the secondary link. The medical data source 202 may respond to such information by adjusting what data it transmits and/or how the data is transmitted. For example, the medical data source 202 may increase the compression of data sent via the primary link to reduce the load on the primary link. Optionally, link quality information may be communicated to the medical data source 202 via a dedicated control link *(i.e.,* a link that is not used for transmitting image data).

As noted above, a medical data source 102 may generate command data that is transmitted to a medical data sink 104 for controlling the medical data sink 104. For example, with reference to FIG. 2, the medical data sink 204 may be configured to perform one or more user-controllable functions, and the medical data source 202 may be configured to receive user input (directly or indirectly) and send command data to the medical data sink 204 to control the one or more user controllable functions. The medical data source 202 may be configured to send command data to the medical data sink 204 via multiple wireless communication links in accordance with the principles described herein.

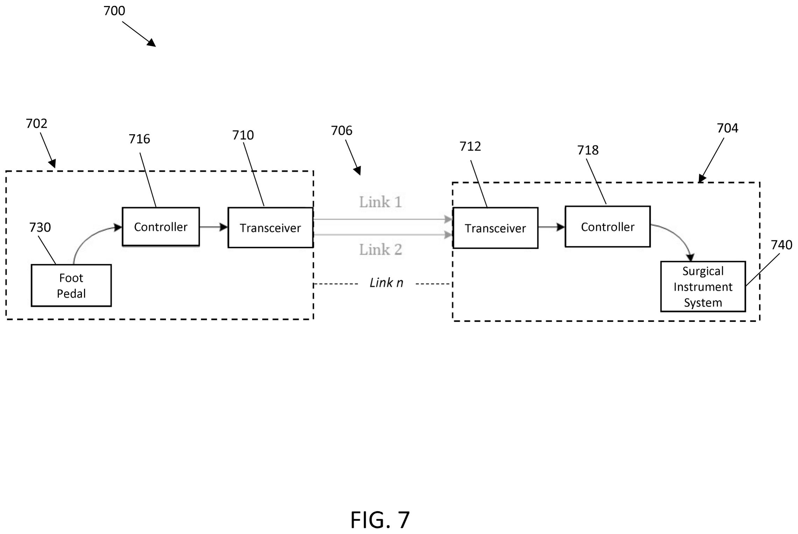

FIG. 7 illustrates an exemplary implementation of system 200 of FIG. 2 in which a medical data source is configured to send command data to a medical data sink for controlling one or more functions of the medical data sink. In the exemplary system 700 of FIG. 7, the medical data source 702 includes a foot pedal 730 (which is an example of medical data generator 214 of system 200) that generates commands based on user actuation (e.g., a press of the foot pedal 730) and the medical data sink 704 includes a surgical instrument system 740 (which is an example of medical data processor 220 of system 200) that performs one or more functions that can be controlled via the foot pedal 730. The foot pedal 730 is merely an exemplary user input device, and it should be understood that any user control device (e.g., a hand-held control device, a voice control device, a touch screen, etc.) can be used. The surgical instrument system 740 can be any system that aids a user in a surgical procedure. The surgical instrument system 740 may be, for example, a powered cutter system for cutting tissue of a subject, a fluid management system for delivering fluid to and/or removing fluid from a surgical site, or a medical imaging system.

The medical data source 702 includes a controller 716 and transceiver 710, which are analogous to controller 216 and transceiver 210 of system 200, respectively. The medical data sink 704 includes a controller 718 and transceiver 712, which are analogous to controller 218 and transceiver 212 of system 200, respectively. The controller 716 of the medical data source 702 is configured to control transmission of medical data (e.g., command data) to the medical data sink 704 via a multi-link wireless communication connection 706 between transceiver 710 of the medical data source 702 and transceiver 712 of the medical data sink 704, according to the principles discussed herein. The controller 718 of the medical data sink 704 is configured to reconstruct the medical data received via the wireless communication connection 706, in accordance with the principles discussed herein. The controller 718 may provide command data received from the medical data source 702 to the surgical instrument system 740, and the surgical instrument system 740 may respond in accordance with the command data. For example, the surgical instrument system 740 may be a cutter system, and the command data may be a command to start or stop a cutting function of a cutter, increase or decrease a speed of the cutting function of the cutter, switch to a different cutting mode of the cutter, or control any other function of the cutter.

In FIG. 7, the foot pedal 730, controller 716, and transceiver 710 of the medical data source 702 represent functional components that can be implemented in a single device or in multiple devices that are communicatively connected. For example, a single device may provide the functionality of the foot pedal 730, the controller 716, and the transceiver 710, or each of the foot pedal 730, the controller 716, and the transceiver 710 can be embodied as a different device. Similarly, the transceiver 712, the controller 718, and the surgical instrument system 740 of the medical data sink 704 of FIG. 7 represent functional components that can be implemented in a single device or in multiple devices that are communicatively connected. For example, a single device may provide the functionality of the surgical instrument system 740, the controller 718, and the transceiver 712, or each of the surgical instrument system 740, the controller 718, and the transceiver 712 can be embodied as a different device.

Using the multi-link wireless communication connection 706 to transmit command data from the foot pedal 730 to the surgical instrument system 740 may improve reliability and/or reduce latency relative to an arrangement that uses a single-link wireless communication connection. Moreover, transmitting the command data over multiple wireless communication links may provide redundancy, thereby increasing reliability of command data transmission. Reliability and latency in command data transmission may be important in ensuring proper control of the surgical instrument system 740 and, thus, patient safety. A given command initiated by the foot pedal 730 may be transmitted by controller 716 and transceiver 710 over at least two separate wireless communication links in accordance with the principles described herein. As discussed above with respect to system 200, the at least two different wireless communication links can include two different channels/frequencies with the same communication protocol (e.g., two different WiFi bands) or two different communication protocols (e.g., WiFi and Bluetooth).

The controller 716 of the medical data source 702 may include unique identifier information in transmissions of command data to the medical data sink 704. The controller 718 of the medical data sink 704 may recombine command data received via the wireless communication connection 706 based on the unique identifier information. For example, the same command data may be sent by controller 716 and transceiver 710 on two links of the wireless communication connection 706, and the controller 718 of the medical data sink 704 may utilize the command data received on one link (e.g., whichever link provides the command data first) and ignore the same command data received on the other link based on a determination that the command data has the same unique identifier information as an already received command data transmission. In some examples, one link is a primary link, and the controller 718 of the medical data sink 704 uses information received on that primary link, unless the controller 718 determines that information has been lost, or otherwise affected, on the primary link, in which case the controller 718 may use information received on one or more secondary links. The controller 718 may determine that information has been lost on the primary link by recognizing unique identifier information on a secondary link that has not been received on the primary link (e.g., within a predetermined timeout).

The unique identification information can include, for example, a serial number, a hash of the command data, a time stamp, or any other information that uniquely identifies a given transmission. In examples in which the command data is transmitted as a single packet, the unique identification information may be the packet itself.

Optionally, medical data source 702 transmits command data and non-command data to the medical data sink 704 via the multi-link wireless communication connection 706. For example, the medical data source 702 may transmit medical images and command data. Optionally, the controller 716 of the medical data source 702 transmits the command data and non-command data according to a reliability requirement. For example, command data may be transmitted according to a first reliability requirement (e.g., a higher-reliability requirement) associated with a first level of tolerance for data loss and/or latency and medical images may be transmitted according to a second reliability requirement (e.g., a lower-reliability requirement relative to the first reliability requirement) associated with a second level of tolerance for data loss and/or latency. Higher-reliability data may be transmitted via multiple links, increasing transmission reliability and/or reducing latency, and lower-reliability data may be sent on only a primary link. The primary link may be a relatively high-bandwidth link (e.g., WiFi) that can normally accommodate both the lower-reliability data and the higher-reliability data, and a secondary link may be a relatively lower-bandwidth link (e.g., Bluetooth) over which only the higher-reliability data (e.g., command data) is transmitted.

The controller 716 of the medical data source 702 and/or the controller 718 of the medical data sink 704 may monitor link quality and adjust use of links accordingly. For example, controller 718 of the medical data sink 704 may determine poor reliability and/or long latency on a given link (e.g., based on packet time stamps and/or packet losses), and in response, may control transceiver 712 to change the channel of the affected link (e.g., change the frequency).

Optionally, a wireless communication connection between a medical data source 702 and a medical data sink 704 may be used for bi-directional communication between the medical data source 702 and the medical data sink 704. For example, the medical data source 702 may send control information to the medical data sink 704, and the medical data sink 704 may provide status information in response.

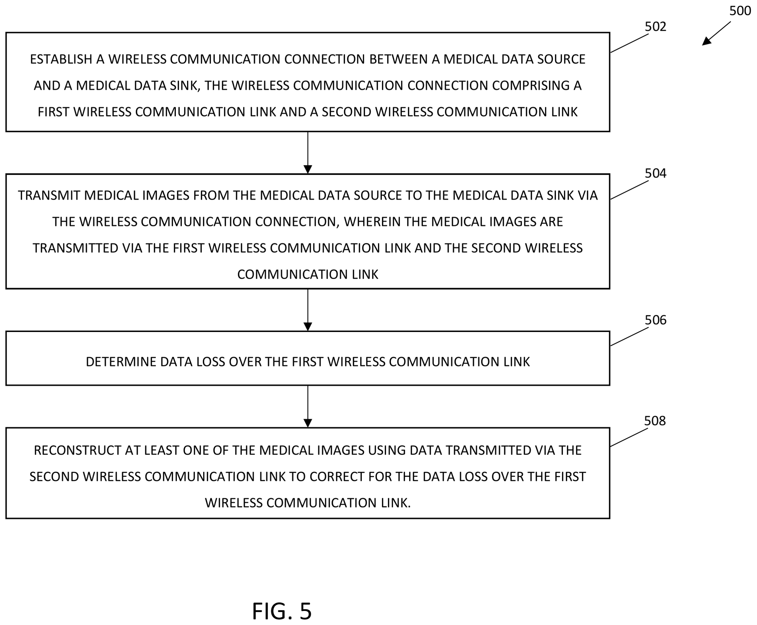

FIG. 5 illustrates an exemplary method 500 for multi-link wireless transmission of medical data. Method 500 may be performed, for example, by any one of the medical data sources 102 and any one of the medical data sinks 104 of FIG. 1 for transmitting medical data from the medical data source 102 to the medical data sink 104. Method 500 may be performed by medical data source 202 and medical data sink 204 of system 200 of FIG. 2 for transmitting medical data, such as medical images (e.g., video frames), non-image information (e.g., system status information), and/or command data. For example, method 500 may be performed by medical data source 702 and medical data sink 704 of system 700 of FIG. 7 for transmitting command data from the medical data source 702 to the medical data sink 704 for use by the medical data sink 704.

At step 502, a wireless communication connection is established between a medical data source and a medical data sink. For example, transceiver 210 of medical data source 202 and wireless transceiver 212 of medical data sink 204 may exchange information to establish a wireless communication connection between them. The wireless communication connection may be a multi-link connection that includes at least two wireless links. The different links may be on different wireless communication bands and/or on different channels of the same band.

Step 502 may include monitoring link quality and adjusting links based on changes in link quality. For example, the wireless transceivers 210 and 212 may change the channel and/or band of a link to improve quality of the link and/or may break one link in favor of a new link that has higher quality. One or both of the medical data source and medical data sink *(e.g.,* wireless transceivers 210 and 212) may monitor various aspects of link quality, such as signal strength, noise, signal-to-noise ratio, channel congestion, data loss, data rate capability, and/or any other information useful for maintaining the wireless communication connection and transmitting data via the wireless communication connection. The medical data source and medical data sink may exchange information for monitoring link quality.

At step 504, medical data may be transmitted from the medical data source to the medical data sink via the wireless communication connection. For example, one or more medical images, non-imaging information, and/or command data may be transmitted from medical data source 202 to medical data sink 204 using links 208A and 208B. Data for a given set of medical data *(e.g.,* a given medical image, given non-imaging information, or given command data) may be transmitted on multiple links. For example, the same medical image (at the same fidelity or at different fidelities) may be transmitted on two different links. For a given set of medical data, the data transmitted via one link may be different than the data transmitted via another link. For example, higher compression may be used on the data sent via one link than the data sent via another link. For the example of a medical image, a higher-fidelity *(e.g.,* higher-resolution) version of the medical image may be transmitted on one link and a lower-fidelity *(e.g.,* lower-resolution) version of the medical image may be transmitted on another link. One of the links may be selected *(e.g.,* by the medical data source 202 alone or in concert with the medical data sink 204) as a primary link based on link quality. The data sent by the medical data source via the primary link may be used by the medical data sink as a primary source for reconstructing the medical data. One or more other links may be designated as a secondary link. The data sent by the medical data source via the secondary link may be used by the medical data sink as a back-up source of data for reconstructing the medical data that may be used to correct for data loss or delay on the primary link. Higher-fidelity information may be transmitted via the primary link and lower-fidelity information may be transmitted via the secondary link.

At least a portion of data for a given set of medical data *(e.g.,* a given medical image) that is transmitted via one of the links may also be transmitted via the other link using the same or different encoding. For example, as mentioned above, duplicates of sets of medical data *(e.g.,* duplicate copies of a medical image, duplicate copies of non-imaging information, or duplicate copies of command data) may be transmitted via both links. Alternatively, data corresponding to more important aspects of the medical data may be transmitted in the same manner via both links and data corresponding to less important aspects of the medical data may be transmitted differently depending on the link. Taking a medical image as an example, the luminance channel of the medical image (which provides more perceivable detail than the chroma channels) may be transmitted with the same fidelity on both a primary link and a secondary link, but the chroma channels may be provided with a higher fidelity on the primary link and with a lower fidelity on the secondary link.

Medical data may be transformed *(e.g.,* by controller 216 of medical data source 202) into higher-fidelity information and lower-fidelity information that can be used in combination to reconstruct the medical data. The lower-fidelity information may be useable by itself for adequately reconstructing the medical data, though not at the quality achievable using both the lower- and higher-fidelity information in combination. Optionally, both the lower-fidelity information and the higher-fidelity information may be transmitted on the primary link but only the lower-fidelity information may be transmitted on the secondary link. The higher-fidelity information may include higher-frequency information for the medical data, information for the medical data compressed with a lower compression ratio, and/or higher-resolution information for the medical data. Conversely, the lower-fidelity information may include lower-frequency information for the medical data, information for the medical data compressed with a higher compression ratio, and/or lower-resolution information for the medical data. In examples in which the medical data is medical images, the higher-fidelity information and/or the lower-fidelity information may include a luminance channel of the medical image and/or one or both chroma channels of the medical image. One or more of the luminance and chroma channels may be decomposed into lower-frequency information (often referred to as a base layer) and higher-frequency information (often referred to as a detail layer). The lower-frequency information, or base layer, may represent smoothly varying aspects of an image, thereby capturing large-scale structural information of an image, whereas the higher-frequency information, or detail layer, may represent small-scale variations of an image, thereby capturing more fine details of an image.

Optionally, data can be redistributed among the links based on link quality. For example, the link that is the primary link and the link that is the secondary link may change depending on which link has higher quality. One or more attributes of the data sent on a given link may be dynamically adjusted based on link quality. For example, a compression ratio used for sending data on a given link may be adjusted higher or lower based on the link quality dropping or increasing, respectively. Additionally, or alternatively, the amount of forward error correction used for sending data on a given link may be dynamically adjusted based on link quality. For example, more forward error correction may be used when the data rate capability of a link increases than when it decreases.

The medical data sink *(e.g.,* controller 218 of medical data sink 204) may reconstruct medical data using the data received via one or more of the links. For example, the medical data sink may reconstruct medical data using the data received via a primary link, which may be higher-fidelity data than the data received via one or more secondary links. The manner in which the medical data is reconstructed may depend on how the medical data was transmitted by the medical data source. For example, if a medical image was converted into higher-fidelity information and lower-fidelity information and the higher- and lower-fidelity information was transmitted on the primary link, reconstructing the medical image by the medical data sink may include reconstructing the lower-fidelity information, reconstructing the higher-fidelity information, and using the lower-fidelity information and the higher-fidelity information to reconstruct the medical image. If a medical image was deconstructed into a base layer and a detail layer, the image may be reconstructed by combining the base and detail layers.

At step 506, data loss or delay over a link is determined. For example, data loss or delay over a primary link may be determined. Data loss or delay over a link may be determined by comparing data received via one link with data received via another link. For example, duplicates of a medical image may be sent via link 208A and 208B, and controller 218 of medical data sink 204 may compare data received via link 208A with data received via link 208B to determine whether at least some data received via link 208B has not been received via link 208A. This comparison may utilize a buffer, such as receive buffer 402 of FIG. 4, that is used to store data received over the links 208 to account for variability in when corresponding data is received over the links 208. At least some of the data in the receive buffer 402 may be checked by processor(s) 404 at a predetermined interval to determine whether data has been lost or delayed over one of the links. In examples in which the medical data is a medical image, the receive buffer *(e.g.,* receive buffer 402) may be large enough for an entire image or may only store a subset of an image, such as a few lines of the image. As noted above, the receive buffer size may be dynamically adjusted to account for changes in link quality. The receive buffer size may be increased *(e.g.,* by processor(s) 404 of FIG. 4) in response to a drop in primary link quality, which may allow additional time for data packets to be received. The receive buffer size may be decreased in response to an increase in primary link quality to decrease latency. Additionally, or alternatively, data loss or delay over a link may be determined based on an expectation of when data should be received. For example, the controller 218 may have some expectation of when data should be received (e.g., based on a pattern of data packet reception frequency) and may determine data loss or delay when the data is not received according to the expectation.

Determining data loss or delay over a link may include analyzing data received via the link for indications of data loss or delay. For example, the data packets received via a link may include error detection attributes that may enable the medical data sink *(e.g.,* controller 218) to determine that one or more packets were not received and/or were not received in full *(e.g.,* by using an error-detecting algorithm such as a cyclic redundancy check). For example, the controller 218 may calculate check values from received data *(e.g.,* checksums), compare the check values to the check values in the received data, and determine that data has been lost or delayed when the check values do not match. Optionally, data packets may include serial numbers associated with the order of the packets and a lost or delayed data packet may be determined by identifying that an expected serial number has not been received. For example, a given data packet having serial number X may be received, a determination may be made that the next data packet should have serial number X+1, and a determination made be made that the next data packet has been lost or delayed if a data packet having serial number X+1 has not been received within an expected time period or a data packet with a higher serial number (e.g., X+2) has been received but not a data packet with serial number X+1.

At step 508, the data loss or delay on a link determined at step 506 is corrected for by reconstructing the medical data using data transmitted via another link. For example, data from a secondary link may be used to replace data lost or delayed via a primary link. Optionally, the medical data is reconstructed entirely from data received via the secondary link even if some of the medical data is successfully received via the primary link. For example, a medical image may be reconstructed from the data received via the secondary link and used instead of reconstructing the image from data received via the primary link, even if some of the data for reconstructing the medical image is received via the primary link. Alternatively, a given set of medical data may be reconstructed in part using data received via the secondary link and data received via the primary link. For example, in reconstructing a medical image, the portion of the medical image affected by the data loss or delay may be the only portion reconstructed using the data received via the secondary link, with the other portions of the medical image being reconstructed using the data received via the primary link. For example, one or more pixels of the medical image, one or more lines of the medical image, one or more regions of the medical image, and/or one or more channels of the medical image and/or one or more channels of one or more regions of an image, may be the only portions of the medical image reconstructed using the data received from the secondary link.

Where the data sent via the primary link and the secondary link differs in fidelity, the medical image or the portion of the medical image that is reconstructed from the data received via the secondary link may have lower fidelity (e.g., lower resolution, lower quality resulting from higher compression, etc.) than it would have had the medical image or portion thereof been reconstructed from the data received via the primary link. The fidelity of the data sent via the secondary link may be sufficient that the difference in fidelity of the reconstrued medical image or portion thereof relative to what it would have been had the medical image been reconstructed entirely from data received via the primary link does not excessively affect a user's *(e.g.,* a surgeon's) ability to adequately visualize the features of interest captured in the medical image. Optionally, any difference is not perceivable to the user.

In some situations, data loss or delay may occur on both a primary link and a secondary link to the extent that a given set of medical data cannot be adequately reconstructed using the data from the primary and secondary links. To handle such situations, method 500 may include determining that a given set of medical data cannot be reconstructed due to data losses or delays over the various links over which it was transmitted (e.g., a primary link and a secondary link) and using a previously received set of data instead. For example, controller 218 of medical data sink 204 may determine that data loss or delay affecting reconstruction of Image N has occurred on both link 208A and link 208B to the extent that Image N cannot be adequately reconstructed (e.g., based on a threshold percentage of pixel data not being received) and/or cannot be adequately reconstructed in time *(e.g.,* in time to display the Image N at a desired video frame rate). Controller 218 may obtain previously reconstructed Image N-1 from image buffer 406 and may provide Image N-1 to display 222 (or otherwise provide the Image N-1 to a downstream function, such as an image processing function).

Upon reconstruction of medical data, the medical data sink (e.g., medical data sink 204 or medical data sink 704) may use the medical data according to the functionality of the medical data sink. For example, the medical data sink may include a display on which a reconstructed medical image and/or reconstructed non-image information (e.g., system status information) is displayed, or the medical data sink may be a surgical instrument system that operates in accordance with reconstructed command data.

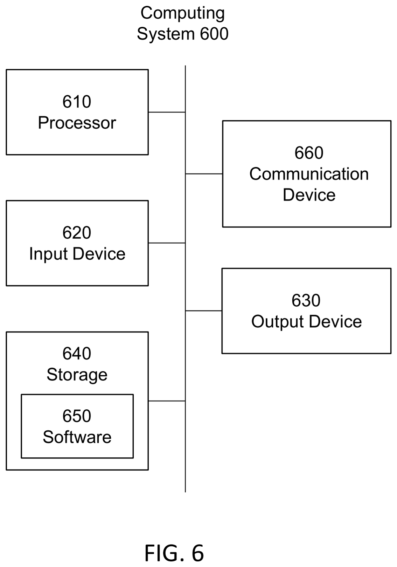

FIG. 6 illustrates an example of a computing system 600 that may be used for any one of the computing systems and devices described herein, such as for any of the medical data sources 102 and medical data sinks 104 of FIG. 1 and/or for the medical data source 202 and/or medical data sink 204 of FIG. 2. System 600 can be a computer connected to a network. System 600 can be a client computer or a server. As shown in FIG. 6, system 600 can be any suitable type of microprocessor-based system, such as a personal computer, workstation, server, or handheld computing device (portable electronic device) such as a phone or tablet. The system can include, for example, one or more of a processor 610, input device 620, output device 630, storage 640, and communication device 660. Input device 620 and output device 630 can generally correspond to those described above and can either be connectable or integrated with the computer.

Input device 620 can be any suitable device that provides input, such as a touch screen, keyboard or keypad, mouse, gesture recognition component of a virtual/augmented reality system, or voice-recognition device. Output device 630 can be or include any suitable device that provides output, such as a touch screen, haptics device, virtual/augmented reality display, or speaker.

Storage 640 can be any suitable device that provides storage, such as an electrical, magnetic, or optical memory, including a RAM, cache, hard drive, removable storage disk, or other non-transitory computer-readable medium. Communication device 660 can include any suitable device capable of transmitting and receiving signals over a network, such as a network interface chip or device. The components of the computer can be connected in any suitable manner, such as via a physical bus or wirelessly.

Software 650, which can be stored in storage 640 and executed by processor 610, can include, for example, the programming that embodies the functionality of the present disclosure (e.g., as embodied in the devices as described above). For example, software 650 can include one or more programs for performing one or more of the steps of method 500.

Software 650 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 640, that can contain or store programming for use by or in connection with an instruction execution system, apparatus, or device.

Software 650 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport-readable medium can include, but is not limited to, an electronic, magnetic, optical, electromagnetic, or infrared wired or wireless propagation medium.

System 600 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 600 can implement any operating system suitable for operating on the network. Software 650 can be written in any suitable programming language, such as C, C++, Java, or Python. In various aspects, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosures of the patents and publications referred to in this application are hereby incorporated herein by reference.

## Claims

1. A method for multi-link wireless transmission of medical images comprising:
establishing a wireless communication connection between a medical data source and a medical data sink, the wireless communication connection comprising a first wireless communication link and a second wireless communication link;
transmitting the medical images from the medical data source to the medical data sink via the wireless communication connection, wherein the medical images are transmitted via the first wireless communication link and the second wireless communication link;
determining data loss over the first wireless communication link; and
reconstructing at least one of the medical images using data transmitted via the second wireless communication link to correct for the data loss over the first wireless communication link.

2. The method of claim 1, wherein at least a portion of data for the medical images that is transmitted via the first wireless communication link is also transmitted via the second wireless communication link.

3. The method of claim 2, wherein determining data loss over the first wireless communication link comprises comparing data received at the medical data sink via the first wireless communication link to data received at the medical data sink via the second wireless communication link.

4. The method of any of the preceding claims, wherein determining data loss over the first wireless communication link comprises analyzing data received via the first wireless communication link for indications of data loss.

5. The method of any of the preceding claims, wherein reconstructing the at least one of the medical images comprises reconstructing a first portion of the at least one of the medical images using data transmitted via the first wireless communication link and reconstructing a second portion of the at least one of the medical images using data transmitted via the second wireless communication link.

6. The method of any of the preceding claims, wherein transmitting medical images from the medical data source to the medical data sink via the wireless communication connection comprises transmitting the medical images at a higher fidelity via the first wireless communication link and transmitting the medical images at a lower fidelity via the second wireless communication link.

7. The method of any of the preceding claims, wherein reconstructing the at least one of the medical images using data transmitted via the second wireless communication link to correct for the data loss over the first wireless communication link comprises using an image transmitted via the second wireless communication link in place of an image affected by the data loss.

8. The method of any of the preceding claims, wherein transmitting the medical images from the medical data source to the medical data sink via the wireless communication connection comprises transforming the medical images into higher fidelity information and lower fidelity information, transmitting both the higher fidelity information and the lower fidelity information via the first wireless communication link, and transmitting the higher fidelity information or the lower fidelity information, but not both, via the second communication link.

9. The method of claim 8, wherein the higher fidelity information comprises higher frequency information, information compressed with a lower compression ratio, and/or higher resolution information, and the lower fidelity information comprises lower frequency information, information compressed with a higher compression ratio, and/or lower resolution information.

10. The method of claim 8 or claim 9, wherein the higher fidelity information comprises at least one of luminance and chroma, and/or wherein the lower fidelity information comprises at least one of luminance and chroma.

11. The method of any of claims 8-10, wherein the lower fidelity information is transmitted via the second communication link.

12. The method of any of the preceding claims, wherein the medical images are video frames, the method optionally comprising determining that a current frame cannot be reconstructed due to data losses over the first and second wireless communication links and retrieving a previous frame from a frame buffer, the method further optionally comprising dynamically adjusting a buffer size based on link quality of the first and second wireless communication links.

13. The method of any of the preceding claims, comprising monitoring link quality of a plurality of wireless communication links that includes the first and second wireless communication links, and switching wireless communication links based on the link quality, and optionally redistributing data among the plurality of wireless communication links based on the link quality.

14. A system for multi-link wireless transmission of medical images, the system comprising:
a medical data source and a medical data sink,
wherein the medical data source comprises one or more processors and memory storing instruction for execution by the one or more processors for causing the medical data source to:
establish a wireless communication connection with the medical data sink, the wireless communication connection comprising a first wireless communication link and a second wireless communication link, and
transmit the medical images from the medical data source to the medical data sink via the wireless communication connection, wherein the medical images are transmitted via the first wireless communication link and the second wireless communication link, and
wherein the medical data sink comprises one or more processors and memory storing instruction for execution by the one or more processors for causing the medical data source to:
determine data loss over the first wireless communication link, and
reconstruct at least one of the medical images using data transmitted via the second wireless communication link to correct for the data loss over the first wireless communication link.

15. A computer program product comprising instructions for execution by one or more processors of a computing system to cause the computing system to:
establish a wireless communication connection between a medical data source and a medical data sink, the wireless communication connection comprising a first wireless communication link and a second wireless communication link;
transmit medical images from the medical data source to the medical data sink via the wireless communication connection, wherein the medical images are transmitted via the first wireless communication link and the second wireless communication link;
determine data loss over the first wireless communication link; and
reconstruct at least one of the medical images using data transmitted via the second wireless communication link to correct for the data loss over the first wireless communication link.
